# EUROPEAN PATENT APPLICATION

(11) **EP 0 573 652 A1**
(43) Date of publication of application: **15.12.1993**
(21) Application number: 92906198.4
(22) Date of filing: 26.02.1992
(51) Int. Cl.: A61K 31/415, A61K 31/535

(54) **REMEDY FOR RENAL DISEASES**

(30) Priority: 01.03.1991 JP 120713/91
(71) Applicant: FUJISAWA PHARMACEUTICAL CO., LTD., Osaka-shi Osaka 541 (JP)
(72) Inventor: ANDOH, Takeshi, Higashinada-ku Kobe-shi Hyogo 658 (JP); NAGATOMI, Itsuo, Yodogawa-ku Osaka-shi Osaka 532 (JP)
(74) Representative: Türk, Gille, Hrabal, Leifert
(86) International application number: JP9200208
(87) International publication number: WO9215296

(57) **Abstract**

A remedy for renal diseases containing an amino acid derivative represented by general formula (I) or its salt as the active ingredient. In the said formula, R¹ represents optionally substituted lower alkyl, etc.; R² represents hydrogen or lower alkyl, or alternatively R¹ and R² form together with the adjacent nitrogen atom an optionally substituted heterocyclic group; R³ represents hydrogen or lower alkyl; and R⁴ represents lower alkyl.

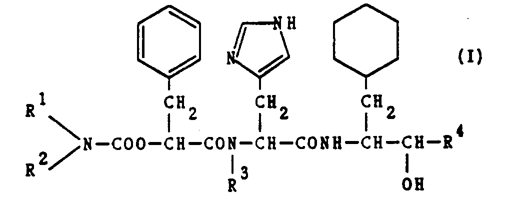

## Description

### Technical Field

This invention relates to an agent for the treatment of renal disorders comprising amino acid derivatives or their salts.

And this invention relates to new use of amino acid derivatives or their salts for the treatment of renal disorders.

More particularly, this invention relates to new use of the amino acid derivatives represented by the general formula (I) given below or their salts for the treatment of renal disorders such as renal failure, diabetic nephropathy, nephritis [e.g. glomerulonephritis, etc.], nephrotic syndrome, renal toxicity induced by a drug, nephredema, gout, hyperuricemia, or the like.

Accordingly, the object of this invention is to provide an agent for the treatment of renal disorders comprising said amino acid derivatives or their salts.

Further, the object of this invention is to provide a pharmaceutical composition for the treatment of renal disorders comprising said amino acid derivatives or their salts as an active ingredient.

Further, the object of this invention is to provide use of said amino acid derivatives or their salts for the manufacture of a medicament for the treatment of renal disorders in human beings or animals.

Still further, the object of this invention is to provide a method for the treatment of renal disorders, which comprises administering said amino acid derivatives or their salts to human beings or animals.

### Background Art

It is known as described in European Patent Application publication No. 300189 that the amino acid derivatives of this invention have renin inhibitory activity and are useful for the treatment of hypertension [e.g. essential hypertension, renal hypertension, malignant hypertension, etc.], heart failure, or the like.

### Disclosure of the Invention

The amino acid derivatives used in this invention can be represented by the following general formula (I). 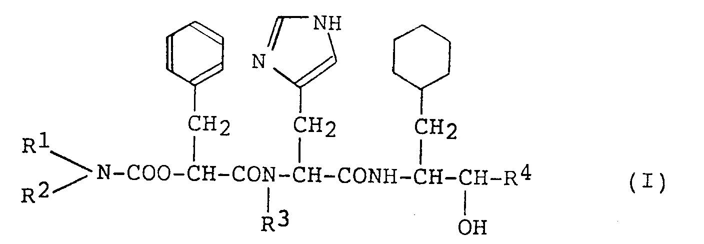
wherein
- R¹ is: lower alkyl optionally substituted with a substituent selected from the group consisting of acyl, hydroxy, lower alkoxy, aryl, lower alkylthio and a group of the formula : 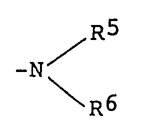 in which
- R⁵ is: hydrogen or acyl and
- R⁶ is: hydrogen or lower alkyl; aryl; or amino optionally substituted with substituent(s) selected from the group consisting of lower alkyl and acyl; and
- R² is: hydrogen or lower alkyl; or
- R¹ and R²: are taken together with the attached nitrogen atom to form a heterocyclic group optionally substituted with substituent(s) selected from the group consisting of lower alkyl, hydroxy(lower)alkyl, lower alkoxy(lower)alkyl, acyl(lower)alkyl, oxo and acyl;
- R³ is: hydrogen or lower alkyl; and
- R⁴ is: lower alkyl.

Particulars of the various definitions mentioned in this specification and claims and preferred examples thereof are explained in the following.

The term "lower" is intended to mean a group having 1 to 7 carbon atom(s), unless otherwise provided.

Suitable "lower alkyl" may be a straight or branched one such as methyl, ethyl, propyl, isopropyl, butyl, isobutyl, tert-butyl, pentyl, isopentyl, hexyl, methylhexyl, heptyl, and the like.

Suitable "acyl" and "acyl" moiety in the term "acyl(lower)alkyl" may be a group of the formula : 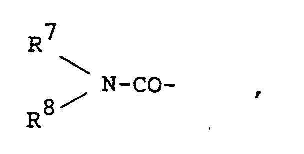
R⁹-CO- and R⁹-SO₂- ,
wherein
- R⁷ and R⁸: are each hydrogen, aryl, cyclo(lower)alkyl, a heterocyclic group or lower alkyl optionally substituted with a substituent selected from the group consisting of lower alkoxycarbonyl, lower alkoxy, aryl and a heterocyclic group, or
- R⁷ and R⁸: are taken together with the attached nitrogen atom to form a heterocyclic group optionally substituted with lower alkyl, and
- R⁹ is: aryl, cyclo(lower)alkyl, lower alkyl optionally substituted with a substituent selected from the group consisting of lower alkoxy and mono- or di(lower)alkylamino, or lower alkoxy optionally substituted with a substituent selected from the group consisting of lower alkanoyl and aryl,
amino-protected or unprotected amino acid residue, or the like.

Suitable "aryl" may be phenyl, naphthyl, tolyl, xylyl, mesityl, cumenyl, and the like, in which preferable one is phenyl.

Suitable "cyclo(lower)alkyl" is cyclopropyl, cyclobutyl, cyclopentyl, cyclohexyl, or the like.

Suitable "heterocyclic group" for R⁷ and R⁸ and one as a substituent on lower alkyl for R⁷ and R⁸ may include saturated or unsaturated, monocyclic or polycyclic one containing at least one hetero atom such as nitrogen atom, oxygen atom or sulfur atom, preferably N, O and/or S containing 5 or 6 membered heterocyclic group, in which the most preferable ones are morpholino, pyridyl and thiazolyl.

Suitable "lower alkoxy" and "lower alkoxy" moiety in the term "lower alkoxycarbonyl" may be a straight or branched one such as methoxy, ethoxy, propoxy, isopropoxy, butoxy, isobutoxy, tert-butoxy, pentyloxy, hexyloxy, and the like, in which more preferable one may be C₁-C₄ alkoxy.

Suitable "heterocyclic group" formed by R⁷, R⁸ and the attached nitrogen atom may be morpholino, thiomorpholino, its 1-oxide or 1,1-dioxide, pyrrolidin-1-yl, pyrazolidin-1-yl, piperidino, piperazin-1-yl, pyrrolin-1-yl, thiazolidin-3-yl, its 1-oxide or 1,1-dioxide, oxazolidin-3-yl, perhydropyridazin-1-yl, 1,4-dihydropyridin-1-yl, 1,2,3,6-tetrahydropyridin-1-yl, 1,2,3,4-tetrahydroisoquinolin-2-yl, 1,2,3,4-tetrahydroquinolin-1-yl, hexamethyleneimino, 1,4-diazabicyclo[4.3.0]nonan-4-yl, and the like.

Suitable "mono- or di(lower)alkylamino" may be methylamino, ethylamino, propylamino, isopropylamino, butylamino, isobutylamino, dimethylamino, methylethylamino, methylisopropylamino, diethylamino, or the like.

Suitable "lower alkanoyl" may be formyl, acetyl, propionyl, butyryl, isobutyryl, valeryl, isovaleryl, pivaloyl, 4-methylvaleryl, or the like.

Suitable "amino-protected or unprotected amino acid residue" may be glycyl, alanyl, β-alanyl, valyl, leucyl, isoleucyl, histidyl, prolyl, seryl, threonyl, cystyl, phenylalanyl, aspartyl, glutamyl, triptophyl, or the like, each amino group of which may be protected by N-protective group such as substituted or unsubstituted lower alkanoyl [e.g. formyl, acetyl, propionyl, trifluoroacetyl, etc.], phthaloyl, lower alkoxycarbonyl [e.g. tert-butoxycarbonyl, tert-amyloxycarbonyl, etc.], substituted or unsubstituted aralkyloxycarbonyl [e.g. benzyloxycarbonyl, p-nitrobenzyloxycarbonyl, etc.], substituted or unsubstituted arenesulfonyl [e.g. benzenesulfonyl, tosyl, etc.], nitrophenylsulfenyl, aralkyl [e.g. trityl, benzyl, etc.] or the like.

Preferred examples of the above-mentioned acyl group may be lower alkanoyl [e.g. formyl, acetyl, propionyl, butyryl, isobutyryl, valeryl, isovaleryl, pivaloyl, 4-methylvaleryl, etc.], mono- or di(lower)alkylamino-(lower)alkanoyl [e.g. methylaminoacetyl, methylaminopropionyl, dimethylaminobutyryl, etc.], lower alkoxy(lower)alkanoyl [e.g. methoxyacetyl, methoxypropionyl, ethoxypropionyl, etc.], aroyl [e.g. benzoyl, toluoyl, etc.], cyclo(lower)alkylcarbonyl [e.g. cyclopropylcarbonyl, cyclobutylcarbonyl, cyclopentylcarbonyl, cyclohexylcarbonyl, etc.], amino-protected or unprotected amino acid residue [e.g. glycyl, benzoylglycyl, t-butoxycarbonylglycyl, t-butoxycarbonylleucyl, acetylleucyl, t-butoxycarbonylhistidyl, etc.], carbamoyl, mono- or di(lower)alkylcarbamoyl [e.g. methylcarbamoyl, ethylcarbamoyl, propylcarbamoyl, isopropylcarbamoyl, butylcarbamoyl, pentylcarbamoyl, isobutylcarbamoyl, tert-butylcarbamoyl, dimethylcarbamoyl, diethylcarbamoyl, methylethylcarbamoyl, methylisopropylcarbamoyl, methylisobutylcarbamoyl, etc.], heterocyclic(lower)alkylcarbamoyl [e.g. picolylcarbamoyl, pyridylethylcarbamoyl, thiazolylmethylcarbamoyl, morpholinomethylcarbamoyl, morpholinoethylcarbamoyl, etc.], N-heterocyclic(lower)alkyl-N-lower alkylcarbamoyl [e.g. N-picolyl-N-methylcarbamoyl, N-pyridylethyl-N-methylcarbamoyl, N-morpholinomethyl-N-ethylcarbamoyl, N-morpholinoethyl-N-methylcarbamoyl, etc.], ar(lower)alkylcarbamoyl [e.g. benzylcarbamoyl, phenethylcarbamoyl, benzhydrylcarbamoyl, etc.], N-ar(lower)alkyl-N-lower alkylcarbamoyl [e.g. N-benzyl-N-methylcarbamoyl, N-phenethyl-N-methylcarbamoyl, N-phenethyl-N-ethylcarbamoyl, etc.], N-aryl-N-lower alkylcarbamoyl [e.g. N-phenyl-N-methylcarbamoyl, etc.], lower alkoxycarbonyl(lower)alkylcarbamoyl [e.g. methoxycarbonylmethylcarbamoyl, ethoxycarbonylmethylcarbamoyl, ethoxycarbonylethylcarbamoyl, etc.], lower alkoxy(lower)alkylcarbamoyl [e.g. methoxymethylcarbamoyl, methoxyethylcarbamoyl, ethoxypropylcarbamoyl, etc.], aroylcarbamoyl [e.g. benzoylcarbamoyl, toluoylcarbamoyl, etc.], heterocycliccarbamoyl [e.g. pyridylcarbamoyl, morpholinocarbamoyl, thiazolylcarbamoyl, etc.], N-heterocyclic-N-lower alkylcarbamoyl [e.g. N-pyridyl-N-methylcarbamoyl, N-thiazolyl-N-methylcarbamoyl, etc.], heterocycliccarbonyl, preferably N-containing heterocyclic-N-ylcarbonyl which may be substituted with lower alkyl [e.g. morpholinocarbonyl, thiomorpholinocarbonyl, piperidinocarbonyl, 4-methyl-1-piperazinylcarbonyl, 1,2,3,6-tetrahydro-1-pyridylcarbonyl, etc.], lower alkoxycarbonyl [e.g. methoxycarbonyl, ethoxycarbonyl, propoxycarbonyl, isopropoxycarbonyl, butoxcarbonyl, isobutoxycarbonyl, tert-butoxycarbonyl, pentyloxycarbonyl, hexyloxycarbonyl, etc.], mono(or di or tri)halo(lower)alkoxycarbonyl [e.g. iodoethoxycarbonyl, dichloroethoxycarbonyl, trichloroethoxycarbonyl, trifluoromethoxycarbonyl, etc.], hydroxy(lower)alkoxycarbonyl [e.g. hydroxymethoxycarbonyl, hydroxyethoxycarbonyl, hydroxypropoxycarbonyl, hydroxybutoxycarbonyl, etc.], ar(lower)alkoxycarbonyl [e.g. benzyloxycarbonyl, phenethyloxycarbonyl, 4-nitrobenzyloxycarbonyl, trityloxycarbonyl, benzhydryloxycarbonyl, etc.], lower alkenyloxycarbonyl [e.g. vinyloxycarbonyl, allyloxycarbonyl, etc.], lower alkanoyl(lower)alkoxycarbonyl [e.g. acetylmethoxycarbonyl, propionylmethoxycarbonyl, acetylethoxycarbonyl, etc.], lower alkylsulfonyl [e.g. mesyl, ethylsulfonyl, propylsulfonyl, isopropylsulfonyl, butylsulfonyl, isobutylsulfonyl, tert-butylsulfonyl, pentylsulfonyl, hexylsulfonyl, etc.], arylsulfonyl [e.g. phenylsulfonyl, tosyl, etc.], or the like.

Suitable "lower alkylthio" may be a straight or branched one such as methylthio, ethylthio, propylthio, isopropylthio, butylthio, isobutylthio, tert-butylthio_{,} pentylthio, hexylthio, and the like, in which more preferable one may be C₁-C₄ alkylthio.

Suitable "heterocyclic group" formed by R¹, R² and the attached nitrogen atom can be referred to the ones formed by R⁷, R⁸ and the attached nitrogen atom as exemplified above.

Suitable "hydroxy(lower)alkyl" may be hydroxymethyl, hydroxyethyl, hydroxypropyl, hydroxyisopropyl, hydroxybutyl, and the like.

Suitable "lower alkoxy(lower)alkyl" may be methoxymethyl, ethoxymethyl, methoxyethyl, ethoxyethyl, methoxypropyl, and the like.

Suitable salts of the compounds (I) are conventional non-toxic salts and include an organic acid addition salt [e.g. formate, acetate, trifluoroacetate, maleate, tartrate, methanesulfonate, benzenesulfonate, toluenesulfonate, etc.], an inorganic acid addition salt [e.g. hydrochloride, hydrobromide, sulfate, phosphate, etc.], a salt with an amino acid [e.g. aspartic acid salt, glutamic acid salt, etc.], or the like.

The prophylactic treatment may be included in the term "treatment".

Preferred embodiments of the Symbols R¹ to R⁴ are as follows :
- R¹ is: lower alkyl substituted with a substituent selected from the group consisting of a group of the formula : 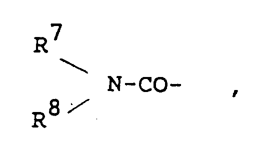 in which
- R⁷ and R⁸: are each hydrogen or lower alkyl, or
- R⁷ and R⁸: are taken together with the attached nitrogen atom to form morpholino,
and a group of the formula : 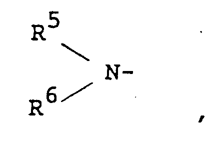 in which
- R⁵ is: hydrogen or a group of the formula : 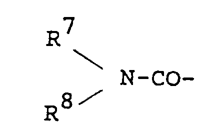 or R⁹-CO-
[in which
- R⁷ and R⁸: are taken together with the attached nitrogen atom to form morpholino, and R⁹ is lower alkyl], and
- R⁶ is: hydrogen or lower alkyl,
- R² is: hydrogen or lower alkyl,
- R³ is: hydrogen or lower alkyl, and
- R⁴ is: lower alkyl.

Particularly, the most interesting compounds are
2(S)-[N^{α}-[2(S)-{N-(2-morpholinocarbonylethyl)-N-methylaminocarbonyloxy}-3-phenylpropionyl}-N^{α}-methyl-L-histidyl]amino-1-cyclohexyl-3(S)-hydroxy-6-methylheptane or its hydrochloride, and
2(S)-[N^{α}-[2(S)-[N-methyl-N-[2-{N-(morpholinocarbonyl)-N-methylamino}ethyl]aminocarbonyloxy]-3-phenylpropionyl]-N^{α}-methyl-L-histidyl]amino-1-cyclohexyl-3(S)-hydroxy-6-methylheptane or its hydrochloride.

It is to be noted that the compound (I) may include one or more stereoisomers due to asymmetric carbon atoms, and all of such isomers and mixture thereof are included within the scope of this invention.

In order to show the usefulness of the compound (I) for the treatment of renal disorders, the pharmacological test data of some representative compounds of the compound (I) are shown in the following.

### Test Compounds

(1) 2(S)-[N^{α}-[2(S)-{N-(2-morpholinocarbonylethyl)-N-methylaminocarbonyloxy}-3-phenylpropionyl]-N^{α}-methyl-L-histidyl]amino-1-cyclohexyl-3(S)-hydroxy-6-methylheptane hydrochloride
(2) 2(S)-[N^{α}-[2(S)-[N-methyl-N-[2-{N-(morpholinocarbonyl)-N-methylamino)ethyl]aminocarbonyloxy]-3-phenylpropionyl]-N^{α}-methyl-L-histidyl]amino-1-cyclohexyl-3(S)-hydroxy-6-methylheptane hydrochloride
(A) Captopril (comparative compound : angiotensin converting enzyme (ACE) inhibitor)

### Test 1

### Effects on increasing renal blood flow

### Method

Male and female cynomolgus monkeys weighing 2.0-4.8 kg were fed with conventional diet were given furosemide 10 mg/kg subcutaneously and 5 mg/kg intravenously on the previous day and the day of dosing with the test compound respectively.

The animals were anesthetized with sodium pentobarbital (20 mg/kg, i.v.) and were maintained at a stable anesthetic level by continuous infusion of the anesthetic at a rate of 6 mg/kg/hr. After fixing the animals on the bench at 37°C, right femoral artery was catheterized for recording of the mean arterial blood pressure using a pressure transducer and left femoral vein was also catheterized for injection of the drug. Left renal artery was installed with an electromagnetic probe for recording renal blood flow using an electromagnetic blood flowmeter. When blood pressure, heart rate and renal blood flow, became sufficiently stable, the test compound dissolved in physiological saline was intravenously given at a rate of 0.2 ml/kg.

### Results

| Test compound | Dose (mg/kg) | Number of animals | Maximum increasing % of renal blood flow | Maximum decreasing % of renal vascular resistance |
|---|---|---|---|---|
| (1) | 0.32 | 5 | +17.1 | +19.3 |
| (2) | 0.32 | 5 | +33.8 | +35.5 |
| (A) | 0.32 | 5 | + 5.7 | +11.5 |

### Test 2

### Diuretic effects

### Method

Female cynomolgus monkeys weighing 3.2 - 3.9 kg were anesthetized with sodium pentobarbital (20 mg/kg, i.v.) and were maintained at a stable anesthetic level by continuous infusion of the anesthetic at a rate of 4 mg/kg/hr, and then right saphenous vein was catheterized for intravenous infusion. Immediately after intravenous injection with paraaminohypuric acid 20 mg/kg and inulin 50 mg/kg, physiological saline containing 0.25% paraaminohypuric acid and 0.1% inulin at a rate of 0.2 ml/kg/min. was intravenously infused. Under monitoring blood pressure, urine samples were taken from bladder by catheter and blood samples were taken from right brachial vein. After 90 min. when urine volume, blood, paraaminohypuric acid and inulin concentrations became stable, blood and urine samples were taken to measure concentrations of paraaminohypuric acid and inulin 45 min. before and 15, 30, 45 and 60 min. after dosing with the test compound. Urine volume and urinary electrolytes were measured and the renal plasma flow and glomerular filtration rate values were calculated from respective paraaminohypuric acid and inulin concentrations in the blood and urine samples.

### Results (Control : 100%)

The following table shows each increasing % at 15 min. after dosing with the test compound.

| Test compound | Dose (mg/kg) | Number of animals | Increasing % of urine volume | Increasing % of Na⁺ excretion | Increasing % of Cl⁻ excretion |
|---|---|---|---|---|---|
| (1) | 3.2 | 5 | 259 | 223 | 417 |
| (2) | 1.0 | 5 | 202 | 205 | 466 |
| (A) | 3.2 | 4 | 118 | 131 | 180 |

| Test compound | Increasing % of K⁺ excretion | Increasing % of renal plasma flow | Increasing % of glomerular filtration rate |
|---|---|---|---|
| (1) | 135 | 149 | 122 |
| (2) | 169 | 169 | 156 |
| (A) | 119 | 110 | 114 |

### Test 3

### Acute toxicity test

### Method

Five rats (Crj:CD(SD) strain) of either sex were used per group. Solutions of the test compound in physiological saline were administered to rats intravenously. The rats were then observed for 14 days. The LD₅₀ values were calculated by the probit method.

### Results

| Test Compound | Sex | LD₅₀ (mg/kg) |
|---|---|---|
| (1) | Male | 81.2 |
| | Female | 93.3 |
| (2) | Male | 55 |
| | Female | 69 |

It is clear from the above results that the compound (I) has activity of increasing renal blood flow and diuretic activity. This means that the compound is useful for the treatment of renal disorders such as renal failure, diabetic nephropathy, nephritis [e.g. glomerulonephritis, etc.], nephrotic syndrome, renal toxicity induced by a drug, nephredema, gout, hyperuricemia, or the like.

The compound (I) of the present invention is also potentially useful for the treatment of other diseases such as hyperaldosteronism, myocardial infarction, cardiomegaly, angina pectoris, Bartter's syndrome, renal tumor, cerebrovascular disorders, central nervous system (CNS) disorders [e.g. Alzheimer's disease, depression, etc.], vascular diseases associated with diabetes [e.g. diabetic neuropathy, diabetic retinopathy, etc.], psoriasis, scleroderma, disorders of intracellular homeostatis, diseases caused by retroviruses [e.g. acquired immunodeficiency syndrome (AIDS), human immunodeficiency virus (HIV) infection, etc.], or the like, and is useful for a diagnostic agent in determining the presence of renin related disorders such as hypertension.

The compound (I) or its salt, which is used as an active ingredient of a pharmaceutical composition, can be administered orally, parenterally or externally (topically) to a mammal including human being in a conventional pharmaceutical preparation such as capsules, micro-capsules, tablets, granules, powders, troches, pills, ointments, suppositories, injection solutions, suspensions, syrups, and the like.

The pharmaceutical preparation mentioned above can be produced by the established procedures using various organic or inorganic carriers, which are conventional for pharmaceutical purpose, such as excipient [e.g. sucrose, starch, mannit, sorbit, lactose, glucose, cellulose, talc, calcium phosphate, calcium carbonate, etc.], binding agent [e.g. cellulose, methyl cellulose, hydroxymethyl cellulose, polypropylpyrrolidone, gelatin, gum arabic, polyethylene glycol, sucrose, starch, etc.], disintegrator [e.g. starch, carboxymethyl cellulose, hydroxypropyl starch, sodium bicarbonate, calcium phosphate, calcium citrate, etc.], lubricant [e.g. magnesium stearate, aerosil, talc, sodium laurylsulfate, etc.], flavoring agent [e.g. citric acid, menthol, glycine, orange powders, etc.], preservative [e.g. sodium benzoate, sodium bisulfite, methylparaben, propylparaben, etc.], stabilizer [e.g. citric acid, sodium citrate, acetic acid, etc.], suspending agent [e.g. methyl cellulose, polyvinylpyrrolidone, aluminum stearate, etc.], dispersing agent [e.g. hydroxypropyimethyl cellulose, etc.], diluting agent [e.g. water, etc.], base wax [e.g. cacao butter, white petrolatum, polyethylene glycol, etc.].

The amount of the active ingredient in such a pharmaceutical preparation may be no more than the amount necessary to produce the desired therapeutic effect. By way of illustration, it can be about 0.2 mg to about 500 mg per unit dose for oral or parenteral administration.

The active ingredient can be administered in a unit dose of 0.1 mg/patient to 500 mg/patient once to 4 times a day. It should be understood that the above dosage may be adjusted according to the patient's age and body weight, the severity of condition and the route and method of administration.

The following Examples are given for the purpose of illustrating this invention in more detail.

### Example 1

| | |
|---|---|
| Test Compound (1) | 5 mg |
| Lactose | 80 mg |

The above-mentioned ingredients were mixed and the mixture was encapsulated to provide the capsule.

### Example 2

| | |
|---|---|
| Test Compound (2) | 5 mg |
| Lactose | 65 mg |

The above-mentioned ingredients were mixed and the mixture was encapsulated to provide the capsule.

## Claims

1. An agent for the treatment of renal disorders comprising a compound of the formula : 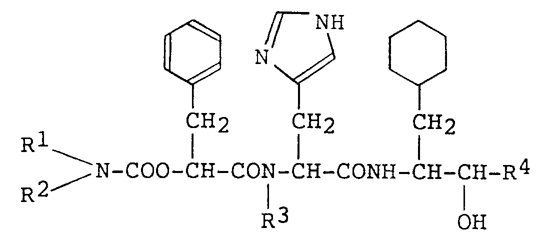 wherein
R¹ is lower alkyl optionally substituted with a substituent selected from the group consisting of acyl, hydroxy, lower alkoxy, aryl, lower alkylthio and a group of the formula : 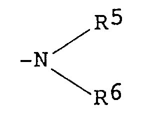 in which
R⁵ is hydrogen or acyl and
R⁶ is hydrogen or lower alkyl; aryl; or amino optionally substituted with substituent(s) selected from the group consisting of lower alkyl and acyl; and
R² is hydrogen or lower alkyl; or
R¹ and R² are taken together with the attached nitrogen atom to form a heterocyclic group optionally substituted with substituent(s) selected from the group consisting of lower alkyl, hydroxy(lower)alkyl, lower alkoxy(lower)alkyl, acyl(lower)alkyl, oxo and acyl;
R³ is hydrogen or lower alkyl; and
R⁴ is lower alkyl;
or its salt as an active ingredient.

2. An agent for the treatment of renal disorders according to claim 1 comprising a compound of claim 1, wherein
R¹ is lower alkyl substituted with a substituent selected from the group consisting of a group of the formula : 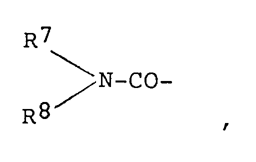 in which
R⁷ and R⁸ are each hydrogen or lower alkyl, or
R⁷ and R⁸ are taken together with the attached nitrogen atom to form morpholino,
and a group of the formula : 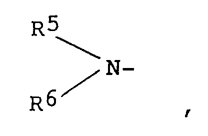 in which
R⁵ is hydrogen or a group of the formula : 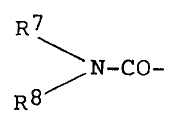 or R⁹-CO-
[in which
R⁷ and R⁸ are taken together with the attached nitrogen atom to form morpholino, and R⁹ is lower alkyl], and
R⁶ is hydrogen or lower alkyl, and
R² is hydrogen or lower alkyl,
as an active ingredient.

3. An agent for the treatment of renal disorders according to claim 2
comprising 2(S)-[N^{α}-[2(S)-{N-(2-morpholinocarbonylethyl)-N-methylaminocarbonyloxy}-3-phenylpropionyl]-N^{α}-methyl-L-histidyl]amino-1-cyclohexyl-3(S)-hydroxy-6-methylheptane or its hydrochloride as an active ingredient.

4. An agent for the treatment of renal disorders according to claim 2
comprising 2(S)-[N^{α}-[2(S)-[N-methyl-N-[2-{N-(morpholinocarbonyl)-N-methylamino}ethyl]aminocarbonyloxy]-3-phenylpropionyl]-N^{α}-methyl-L-histidyl]amino-1-cyclohexyl-3(S)-hydroxy-6-methylheptane or its hydrochloride as an active ingredient.

5. A pharmaceutical composition for the treatment of renal disorders comprising a compound of the formula : 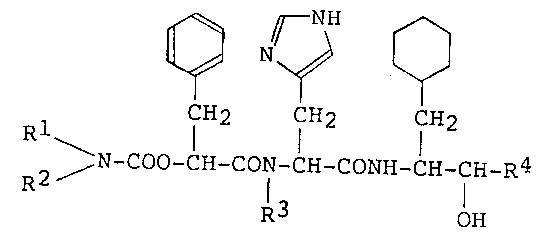 wherein
R¹ is lower alkyl optionally substituted with a substituent selected from the group consisting of acyl, hydroxy, lower alkoxy, aryl, lower alkylthio and a group of the formula : 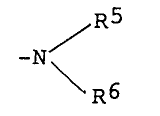 in which
R⁵ is hydrogen or acyl and
R⁶ is hydrogen or lower alkyl; aryl; or amino optionally substituted with substituent(s) selected from the group consisting of lower alkyl and acyl; and
R² is hydrogen or lower alkyl; or
R¹ and R² are taken together with the attached nitrogen atom to form a heterocyclic group optionally substituted with substituent(s) selected from the group consisting of lower alkyl, hydroxy(lower)alkyl, lower alkoxy(lower)alkyl, acyl(lower)alkyl, oxo and acyl;
R³ is hydrogen or lower alkyl; and
R⁴ is lower alkyl;
or its salt as an active ingredient, in association with a pharmaceutically acceptable, substantially nontoxic carrier or excipient.

6. A pharmaceutical composition according to claim 5 comprising a compound of claim 5, wherein
R¹ is lower alkyl substituted with a substituent selected from the group consisting of a group of the formula : 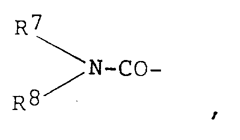 in which
R⁷ and R⁸ are each hydrogen or lower alkyl, or
R⁷ and R⁸ are taken together with the attached nitrogen atom to form morpholino,
and a group of the formula : 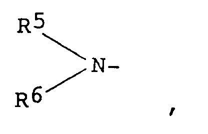 in which
R⁵ is hydrogen or a group of the formula : 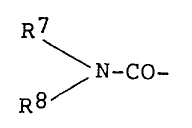 or R⁹-CO-
[in which
R⁷ and R⁸ are taken together with the attached nitrogen atom to form morpholino, and R⁹ is lower alkyl], and
R⁶ is hydrogen or lower alkyl, and
R² is hydrogen or lower alkyl,
as an active ingredient.

7. A pharmaceutical composition according to claim 6 comprising 2(S)-[N^{α}-[2(S)-{N-(2-morpholinocarbonylethyl)-N-methylaminocarbonyloxy}-3-phenylpropionyl]-N^{α}-methyl-L-histidyl]amino-1-cyclohexyl-3(S)-hydroxy-6-methylheptane or its hydrochloride as an active ingredient.

8. A pharmaceutical composition according to claim 6 comprising 2(S)-[N^{α}-[2(S)-[N-methyl-N-[2-{N-(morpholinocarbonyl)-N-methylamino}ethyl]aminocarbonyloxy]-3-phenylpropionyl]-N^{α}-methyl-L-histidyl]amino-1-cyclohexyl-3(S)-hydroxy-6-methylheptane or its hydrochloride as an active ingredient.

9. Use of a compound of the formula : 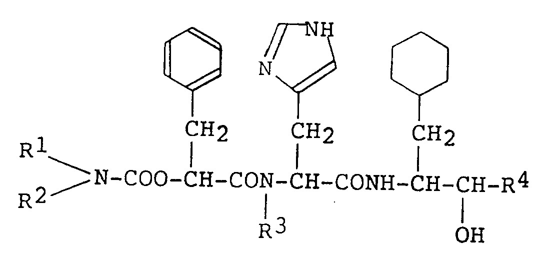 wherein
R¹ is lower alkyl optionally substituted with a substituent selected from the group consisting of acyl, hydroxy, lower alkoxy, aryl, lower alkylthio and a group of the formula : 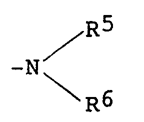 in which
R⁵ is hydrogen or acyl and
R⁶ is hydrogen or lower alkyl; aryl; or amino optionally substituted with substituent(s) selected from the group consisting of lower alkyl and acyl; and
R² is hydrogen or lower alkyl; or
R¹ and R² are taken together with the attached nitrogen atom to form a heterocyclic group optionally substituted with substituent(s) selected from the group consisting of lower alkyl, hydroxy(lower)alkyl, lower alkoxy(lower)alkyl, acyl(lower)alkyl, oxo and acyl;
R³ is hydrogen or lower alkyl; and
R⁴ is lower alkyl;
or its salt for the manufacture of a medicament for the treatment of renal disorders in human beings or animals.

10. Use according to claim 9 of a compound of claim 9, wherein
R¹ is lower alkyl substituted with a substituent selected from the group consisting of a group of the formula : 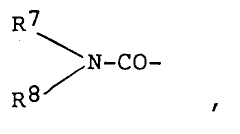 in which
R⁷ and R⁸ are each hydrogen or lower alkyl, or
R⁷ and R⁸ are taken together with the attached nitrogen atom to form morpholino,
and a group of the formula : 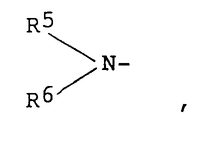 in which
R⁵ is hydrogen or a group of the formula : 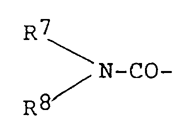 or R⁹-CO-
[in which
R⁷ and R⁸ are taken together with the attached nitrogen atom to form morpholino, and R⁹ is lower alkyl], and
R⁶ is hydrogen or lower alkyl, and
R² is hydrogen or lower alkyl.

11. Use according to claim 10 of 2(S)-[N^{α}-[2(S)-[N-(2-morpholinocarbonylethyl)-N-methylaminocarbonyloxy}-3-phenylpropionyl]-N^{α}-methyl-L-histidyl]amino-1-cyclohexyl-3(S)-hydroxy-6-methylheptane or its hydrochloride.

12. Use according to claim 10 of 2(S)-[N^{α}-[2(S)-[N-methyl-N-[2-{N-(morpholinocarbonyl)-N-methylamino}ethyl]aminocarbonyloxy]-3-phenylpropionyl]-N^{α}-methyl-L-histidyl]amino-1-cyclohexyl-3(S)-hydroxy-6-methylheptane or its hydrochloride.

13. A method for the treatment of renal disorders which comprises administering a compound of the formula : 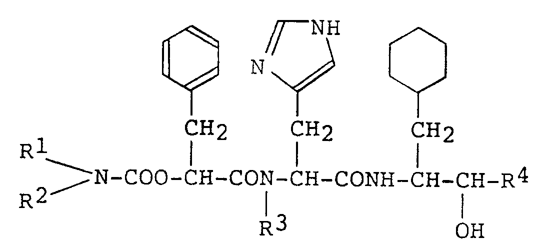 wherein
R¹ is lower alkyl optionally substituted with a substituent selected from the group consisting of acyl, hydroxy, lower alkoxy, aryl, lower alkylthio and a group of the formula : 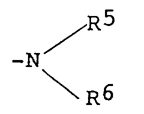 in which
R⁵ is hydrogen or acyl and
R⁶ is hydrogen or lower alkyl; aryl; or amino optionally substituted with substituent(s) selected from the group consisting of lower alkyl and acyl; and
R² is hydrogen or lower alkyl; or
R¹ and R² are taken together with the attached nitrogen atom to form a heterocyclic group optionally substituted with substituent(s) selected from the group consisting of lower alkyl, hydroxy(lower)alkyl, lower alkoxy(lower)alkyl, acyl(lower)alkyl, oxo and acyl;
R³ is hydrogen or lower alkyl; and
R⁴ is lower alkyl;
or its salt to human beings or animals.

14. A method according to claim 13 which comprises administering a compound of claim 13, wherein
R¹ is lower alkyl substituted with a substituent selected from the group consisting of a group of the formula : 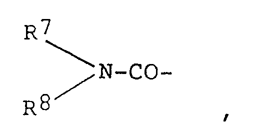 in which
R⁷ and R⁸ are each hydrogen or lower alkyl, or
R⁷ and R⁸ are taken together with the attached nitrogen atom to form morpholino,
and a group of the formula : 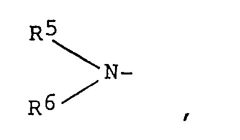 in which
R⁵ is hydrogen or a group of the formula : 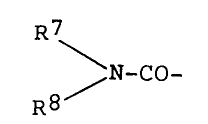 or R⁹-CO-
[in which
R⁷ and R⁸ are taken together with the attached nitrogen atom to form morpholino, and R⁹ is lower alkyl], and
R⁶ is hydrogen or lower alkyl, and
R² is hydrogen or lower alkyl.

15. A method according to claim 14 which comprises administering 2(S)-[N^{α}-[2(S)-{N-(2-morpholinocarbonylethyl)-N-methylaminocarbonyloxy}-3-phenylpropionyl]-N^{α}-methyl-L-histidyl]amino-1-cyclohexyl-3(S)-hydroxy-6-methylheptane or its hydrochloride.

16. A method according to claim 14 which comprises administering 2(S)-[N^{α}-[2(S)-[N-methyl-N-[2-{N-(morpholinocarbonyl)-N-methylamino}ethyl]aminocarbonyloxy]-3-phenylpropionyl]-N^{α}-methyl-L-histidyl]amino-1-cyclohexyl-3(S)-hydroxy-6-methylheptane or its hydrochloride.
